(19) Europäisches Patentamt
European Patent Office
Office européen des brevets

(11) Publication number: 0 037 167 B1

(12) # EUROPEAN PATENT SPECIFICATION

(45) Date of publication of patent specification: 14.12.83

(51) Int. Cl.³: C 07 C 179/04, C 07 C 179/053, C 07 C 178/00, B 01 J 31/02

(21) Application number: 81300654.1

(22) Date of filing: 18.02.81

(54) **Manufacture of hydroperoxides.**

(30) Priority: 31.03.80 US 135306

(43) Date of publication of application: 07.10.81 Bulletin 81/40

(45) Publication of the grant of the patent: 14.12.83 Bulletin 83/50

(84) Designated Contracting States: BE CH DE FR GB IT LI LU NL SE

(56) References cited:
GB - A - 713 138
US - A - 3 428 690

(73) Proprietor: THE STANDARD OIL COMPANY
Midland Building
Cleveland, Ohio 44115 (US)

(72) Inventor: Sundeen, Marcia Henniger
2165 Rexwood Road
Cleveland Heights Ohio 44118 (US)
Inventor: Velenyi, Louis Joseph
1266 Roland Road
Lyndhurst Ohio 44124 (US)
Inventor: Dolhyj, Serge Roman
1520 David Avenue
Parma Ohio 44134 (US)

(74) Representative: Smith, Sydney et al,
Elkington and Fife High Holborn House 52/54
High Holborn
London WC1V 6SH (GB)

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 037 167 B1

# Manufacture of hydroperoxides

This invention relates to the manufacture of hydroperoxides utilising particular catalysts. More particularly it relates to the catalytic manufacture of aryl hydroperoxides from corresponding aryl compounds utilising particular catalysts.

The oxidation of aryl compounds to form aryl hydroperoxides is known generally. For example, the first step in a widely practiced process for manufacturing phenol is the oxidation of cumene to cumene hydroperoxide and was first demonstrated by Hock and Lang, *Chem Ber., 77B*, 257 in 1944. Since that time many improvements have been made as illustrated by USP 2,547,938 (use of base, emulsifying agent and initiator), USP 2,973,310 (use of hydrogen bromide or a bromine salt as a catalyst), USP 3,290,384 (use of alkali or alkaline earth metal salt of an oxy acid of plumbate, bismuthate, stannate or antimonate,) USP 3,959,281 (using cumene hydroperoxide as an initiator), USP 4,022,841 (using organometallic complexes as catalyst), and USP, 4,034,047 (using polyacrylonitrile as a catalyst).

As is evident from the preceding, much of the past research effort has been directed to the identification and development of useful hydroperoxidative catalysts. However, many prior art catalysts are not truly catalysts but are merely vehicles which provide favorable conditions for a hydroperoxidative reaction. For example, the addition of base to the reaction mixture removes the organic acids formed during reaction which, if allowed to remain, would decompose the hydroperoxidation products resulting in unwanted by-products. As another example, emulsifiers merely increase the surface area between the gaseous oxygen and the liquid aryl hydrocarbon reactant. Other examples can be cited. As a consequence, the class of materials that are effective hydroperoxidative catalyst is not extensive and is limited generally to phthalocyanines (USP 3,873,625), organometallic compounds (USP, 3,290,384), inorganic compounds (zinc oxide, lead oxide, magnesium oxide, etc), and certain polymers (USP 4,034,047).

In USP 3,160,668 there is disclosed the use of primary amines having a tertiary alkyl group attached to the amino nitrogen atom in the oxidation of aralkyl hydrocarbons to aralkyl hydroperoxides, such as cumene to cumene hydroperoxide. The amines in question include mixtures of amines having the formula

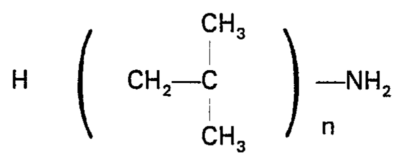

wherein n is 3 to 6. There is no disclosure of the use of initiators with these amines. Included in the disclosure, by way of contrast, is an Example using octadecylamine as the amine.

In GB—PS—713138 there is disclosed a process for the production of an aralkyl hydroperoxide by the reaction of the corresponding aralkyl hydrocarbon with an oxygen containing gas, which reaction is carried out in the presence of a secondary or tertiary amine. Such amine can be a heterocyclic nitrogen compound such as pyridine. There is not disclosure of polymeric secondary amines.

We have now found that straight chain $C_{14}$—$C_{16}$ primary alkyl amines and polyvinyl-pyrrolidones having a weight average molecular weight of at leat 1,000 when used with an initiator demonstrate both improved activity and excellent selectivity in the catalytic hydroperoxidation of aryl compounds. Such catalyst and initiator combinations can also be used for the hydroperoxidation of cycloaliphatic-substituted aryl compounds, such as cyclohexylbenzene.

According to the invention therefore there is provided a process for manufacturing an aryl hydroperoxide of the formula

$$Ar—(R'—OOH)_n \qquad (I)$$

from a corresponding aryl compound of the formula

$$Ar—(R)_m \qquad (II)$$

where

Ar is an aryl radical and each R is independently hydrogen or a radical of the formula

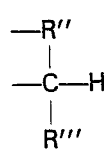 or 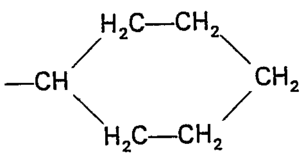

each R' is a radical of the formula

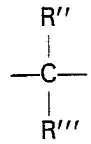 or 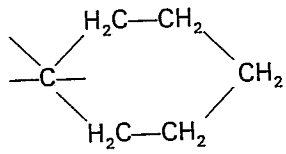

R'' and R''' are independently hydrogen or alkyl radicals of 1 to 3 carbon atoms with the proviso that both cannot be simultaneously hydrogen,

m is 1 or 2 with the provisos that when m is 1, R is not hydrogen, and when m is 1, at least one R is not hydrogen, and

n is 1 or 2,

wherein the corresponding aryl compound (II) is contacted with molecular oxygen at hydroperoxidative conditions in the presence of an initiator and a catalyst selected from a $C_{14}$—$C_{16}$ straight chain primary alkyl amine and poly(vinyl)pyrrolidones having a weight average molecular weight of at least 1,000.

The invention will now be further described by reference to the catalysts, aryl compounds etc.

Catalysts:

Those straight chain primary alkyl amines that are employed in the process of this invention contain 14 to 16 carbon atoms. Examples of amines which may be used include hexadecylamine, octadecylamine and dodecylamine. 1-Hexadecylamine is a preferred primary amine.

The polyvinylpyrrolidones that can be used in this invention as catalysts are known polymers (Kirk-Othmer, *Encyclopedia of Chemical Technology, 17*, 405, 1967) prepared by the addition polymerization of N-vinyl-2-pyrrolidone. These polymers are available commercially and range in weight average molecular weight of 1,000 to 500,000, with those in the range of 10,000 to 100,000 being preferred. Depending upon the reaction conditions and molecular weight of the polymer, the polyvinylpyrrolidone catalysts can be either homogeneous (solubilized or heterogeneous (partially or essentially completely unsolubilized).

Reagents:

The products of the invention are aryl hydroperoxides of the formula

$$Ar—(R'—OOH)_n \qquad (I)$$

and are made by contacting a corresponding aryl compound of the formula

$$Ar—(R)_m \qquad (II)$$

where

Ar is an aryl radical, and each R is independently hydrogen or a radical of the formula

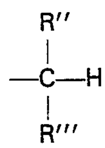 or 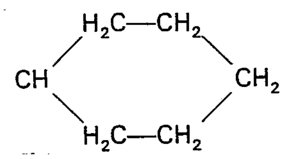

each R' is a radical of the formula

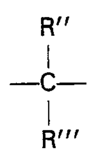 or 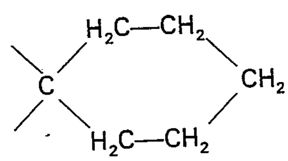

R'' and R''' are independently hydrogen or alkyl radicals of 1 to 3 carbon atoms with the proviso that both cannot simultaneously be hydrogen,

m is 1 or 2 with the provisoes that when m is 1, R is not hydrogen, and when m is 2 at least one R is not hydrogen, and

n is 1 or 2.

Representatives compounds of formula II include ethylbenzene, cumene, cyclohexylbenzene, bicyclohexylbenzene, diisopropylbenzene, 2-phenylbutane, 3-phenylhexane and p-cyclohexylisopropylbenzene. The representative hydroperoxides of formula I include ethylbenzene hydroperoxide, cumene hydroperoxide, cyclohexylbenzene hydroperoxide, bicyclohexylbenzene dihydroperoxide, 2-phenylbutane hydroperoxide and 3-phenylhexane hydroperoxide. The invention is particularly useful for the production of cumene hydroperoxide from cumene and cyclohexylbenzene

hydroperoxide from cyclohexylbenzene, these reactions being graphically depicted as III and IV respectively.

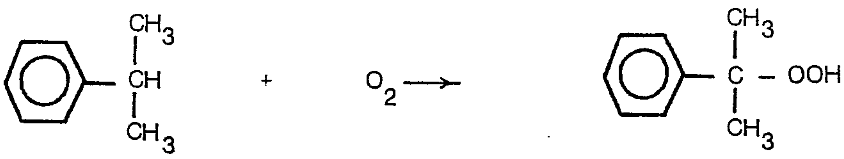

(III)

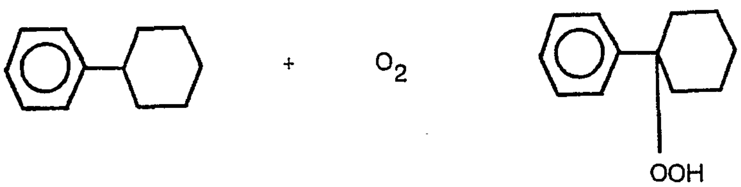

The oxygen required for this reaction is usually molecular oxygen. Either pure or diluted oxygen can be used with air being the most typical and convenient form and source of the oxygen employed.

Process Conditions

Hydroperoxidative conditions are used. These conditions are defined as those at which the compounds of II can be oxidized with molecular oxygen to the compounds of I in the presence of either the primary amines or polyvinylpyrrolidone previously described. In general, the temperature ranges from 90° to 130°C and preferably 100° to 120°C. Temperatures less than 90°C and greater than 130°C can be employed but usually to no advantage.

Atmospheric, subatmospheric or superatmospheric pressure can be employed, the pressure having a relationship to the temperature and usually determined by matters of convenience and economy. Pressures in excess at atmospheric pressure, typically up to 25 atmospheres, are preferred since such pressures favour hydroperoxide formation and thus tend to enhance product yield.

While the reaction time can vary widely, the longer the reaction mixture is held at elevated temperatures, the more product can decompose. Preferably therefore the reaction is conducted only for a sufficient time to reach optimum hydroperoxide yield and then terminated. A reaction time of 180 minutes is a typical minimum while the reaction time of about one day is a typical maximum. Optimum times are dependent upon many variables including the nature and form of the reagents and catalysts temperature, etc.

Solvents are necessary in carrying out the reaction invention only to the extent that the starting material is a solid at reaction conditions. If the aryl compounds are liquid at such conditions, then the reaction can be conducted neat that is without solvent. If a solvent is employed, then it is one that is essentially inert to the reaction reagents, catalysts and products at reaction conditions, and examples of such solvents include the various aliphatic hydrocarbons, chlorinated aromatics and halogenated hydrocarbons. These solvents are liquid at reaction conditions and preferably have a boiling point in excess of the operating temperature of the reaction, n-Heptane, toluene, n-octane, chlorolbenzene, p-xylene and pyridine are all examples of suitable solvents.

Like the other reaction parameters, the relative amounts of reagents and catalysts employed in this invention are not critical. Obviously, the reaction requires stoichiometric amounts of aryl compound and oxygen, the exact amounts dependent upon the number of hydroperoxizable sites on the aryl compounds (when n is 1, the ratio of II to oxygen is 1 and when n is 2, the ratio of II to oxygen is 1:2). From the point of view of both economy and convenience, the reaction is performed in an excess of oxygen.

Sufficient primary amine or polyvinylpyrrolidone to catalyze the reaction is employed. Typically, this amount is at least about 0.05 weight percent, based upon the weight of the aryl compound, and preferably at least about 1 weight percent. While amounts in excess of 10 weight percent can be employed, such amounts usually afford no advantage to the invention.

Other compounds that can be present in the reaction mixture include bases and emulsifiers. Bases, such as hydroxides and carbonates, are useful for maintaining a state of alkalinity which in turn suppress the formation of unwanted by-products. Emulsifiers enhance the surface are between the aryl compound and molecular oxygen thus providing a more efficient reaction. Initiators which are present in the reaction mixture include compounds such as alkyl- and cycloalkyl-substituted aryl hydroperoxide. Preferably, the initiator is the same as the desired hydroperoxidation product, e.g. in cumene hydroperoxidation, cumene hydroperoxide is the initiator. In commercial operation part of the product can be recycled for this purpose.

Products:

The products of this invention are made by the hydroperoxidation of the corresponding aryl compounds e.g. cumene hydroperoxide is made from the peroxidation of cumene and cyclohexylbenzene hydroperoxide is made from the peroxidation of cyclohexylbenzene. Hydroperoxidation will occur at the secondary or tertiary carbon adjoining the aromatic nucleuc and if the aromatic compound has more than 1 such carbon then hydroperoxidation will occur at both such carbons. The products are useful intermediates in the production of phenol and dihydroxybenzen, both manufactured by the acid cleavage of cumene hydroperoxide forms phenol and acetone while the acid cleavage of cyclohexylbenzene hydroperoxide forms phenol and cyclohexanone.

The following examples are illustrate certain embodiments of this invention. Unless indicated to the contrary, all parts and percentages are by weight.

SPECIFIC EMBODIMENTS

Procedure:

Examples 1—7 and controls A—L were performed at atmospheric pressure with cyclohexylbenzene as the aryl compound and molecular hexylbenzene as the aryl compound and molecular oxygen as the peroxidation agent. Cumene hydroperoxide was used as an initiator in each run. Some runs employed n-octane as a solvent others toluene and yet others were performed neat that is the absence of a solvent. 50 grams of cyclohexylbenzene, 0.5 grams of catalyst and 0.5 grams of initiator were used. The catalyst, cyclohexylbenzene, cumene hydroperoxide and optionally the solvent were placed in a round-bottom 3-neck flask fitted with a condenser, thermometer and a stainless steel oxygen inlet tube. The flask was heated on a heating mantel with continuous stirring while the oxygen was sparged through the liquid at a typical flow rate of about 90 cc/min. Samples were taken periodically and titrated with sodium thiosulfate to determine the percentage of cyclohexylbenzene hydroperoxide formed. The experiments were conducted at 115°C and the results with various catalysts, both within and without this invention, are reported in Table 1.

Hydroperoxidation of Cyclohexylbenzene to Cyclohexylbenzene Hydroperoxide

| EXAMPLE | CATALYST | SOLVENT | CONVERSION OF CBX TO CBXHPO[1] | REACTION TIME |
|---|---|---|---|---|
| 1 | PVP[2] | n-Octane | 23.3 | 24 hours |
| 2 | 1-Octadecyl amine | " | 12.2 | " |
| 3 | 1-Octadecyl amine | — | 3.6 | 4.5 hours |
| 4 | 1-Hexadecyl amine | — | 4.4 | " |
| 5 | 1-Dodecyl amine | — | 2.9 | " |
| 6 | 1-Octylamine | — | 3.0 | " |
| 7 | 1-Hexylamine | — | 2.8 | " |
| CONTROL | | | | |
| A | PVP/PS[3] | n-Octane | 5.1 | 24 hours |
| B | PBA[4] | " | 1.7 | " |
| C | Diethylene Triamine | Toluene | 0 | " |
| D | Tributyl amine | " | 0 | " |
| E | Stearamide | " | 6.7 | " |
| F | Piperazine | n-Octane | 0 | " |
| G | Butylamine | — | 0.6 | 4.5 hours |
| H | Dibenzylamine | — | 0.3 | " |
| I | Melamine | n-Octane | 5.7 | 24 hours |
| J | N-Benzylidene Methylamine | " | 1.8 | " |
| K | Phenylene Diamine | " | 0 | " |
| L | Dicyclohexyl amine | " | 0 | " |

$$^{1}\text{Conversion} = \frac{\text{Cyclohexylbenzene hydroperoxide}}{\text{Cyclohexylbenzene reacted}} \times 100$$

[2]Poly(vinyl)pyrrolidone of about 40,000 weight average molecular weight.
[3]Poly(vinyl)pyridine/polystyrene
[4]Polybenzylamine

As is evidenced by these tabulated results, the process of the invention shows good results for the production of aryl hydroperoxide as compared to processes employing as catalysts materials other than those used according to the invention.

Examples 8 and 9

The procedure of Examples 1—7 and Controls A—L was repeated except cumene was substituted for cyclohexylbenzene, superatmospheric pressure was substituted for atmospheric pressure, and no solvent was used. The catalysts and results are reported in Table II.

TABLE II
Hydroperoxidation of Cumene to Cumene Hydroperoxide

| EXAMPLE | CATALYST | PRESSURE (psig) | CONVERSION[1] | REACTION TIME |
|---|---|---|---|---|
| 8 | PVP[2] | 200(14 kg/cm²) | 20.0 | 4.25 hours |
| 9 | 1-Hexadecyl amine | 280 (19.6 kg/cm²) | 20.5 | 3.5 hours |

$$^{1}\text{Conversion} = \frac{\text{Cyclohexylbenzene hydroperoxide products}}{\text{Cyclohexylbenzene reacted}} \times 100$$

[2]Poly(vinyl)pyrrolidone of about 40,000 weight average molecular weight.

**Claims**

1. A process for manufacturing an aryl hydroperoxide of the formula

$$Ar\text{—}(R'\text{—}OOH)_n \qquad (I)$$

from a corresponding aryl compound of the formula

$$Ar\text{—}(R)_m \qquad (II)$$

where

Ar is an aryl radical and each R is independently hydrogen or a radical of the formula

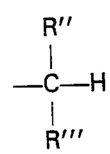

or

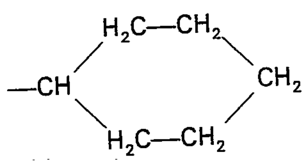

each R′ is a radical of the formula

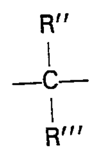

or

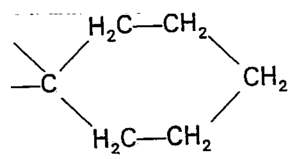

R″ and R‴ are independently hydrogen or alkyl radicals of 1 to 3 carbon atoms with the proviso that both cannot be simultaneously hydrogen,

m is 1 or 2 with the provisoes that when m is 1, R is not hydrogen, and when m is 2, at least one R is not hydrogen, and

n is 1 or 2,

wherein the corresponding aryl compound (II) is contacted with molecular oxygen at hydroperoxidative conditions in the presence of an initiator and a catalyst characterised in that the catalyst is selected from a $C_{14}$—$C_{16}$ straight chain primary alkyl amine and poly(vinyl)pyrrolidones having a weight average molecular weight of at least 1,000.

2. A process as claimed in claim 1 characterised in that the poly(vinyl)pyrrolidone has a weight average molecular weight between 10,000 and 100,000.

3. A process as claimed in claim 1 or claim 2 characterised in that R'' and R''' are methyl radicals.

4. A process as claimed in any of claims 1 to 3 characterised in that a temperature within the range of from 90° to 130°C is employed.

5. A process as claimed in any of claims 1 to 4 characterised in that atmospheric or superatmospheric pressure is employed.

6. A process as claimed in any of claims 1 to 5 characterised in that the catalyst is present in at least 0.05 weight percent based upon the weight of the aryl compound of formula II.

7. A process as claimed in any of claims 1 to 6 characterised in that the aryl compound of formula II is cumene or cyclohexylbenzene.

8. A process as claimed in any of claims 1 to 7 characterised in that the catalyst is 1-hexadecylamine or poly(vinyl)pyrrolidone having a weight average molecular weight of 40,000.

9. A process as claimed in any of claims 1 to 8 characterised in that the initiator is an alkyl or cycloalkyl-substituted aryl hydroperoxide.

10. A process as claimed in claim 9 characterised in that the aryl hydroperoxide is an aryl hydroperoxide of formula I defined in claim 1.

11. A process as claimed in claim 10 characterised in that the initiator is cumene hydroperoxide and the compound of formula II defined in claim 1 is cumene.

**Revendications**

1. Procédé de fabrication d'un hydroperoxyde d'aryle de formule:

$$Ar—(R'—OOH)_n \qquad (I)$$

à partir d'un composé arylé correspondant de formule:

$$Ar—(R)_m \qquad (II)$$

formules dans lesquelles

Ar est un radical aryle et chaque R est indépendamment l'hydrogène où un radical de formule

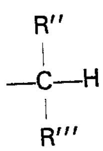

ou

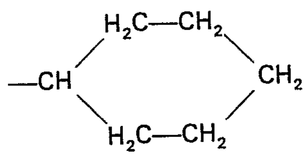

chaque R' est un radical de formule:

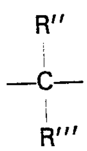

ou

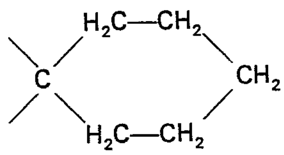

R'' et R''' représentent indépendamment l'hydrogène ou des radicaux alkyles ayant de 1 à 3 atomes de carbone, à la condition que tous deux ne puissent représenter simultanément de l'hydrogène,

m est 1 ou 2, avec les conditions que quand m est 1, R ne représente pad de l'hydrogène, et quand m est 2, au moins un R ne représente pas de l'hydrogène, et quand m est 2, au moins un R ne représente pas de l'hydrogène, et

n est 1 ou 2,

procédé suivant lequel on met en contact le composé arylé correspondant (II) avec de l'oxygène moléculaire, dans des conditions hydroperoxydantes, en présence d'un initiateur et d'un catalyseur, caractérisé par le fait qu'on choisit le catalyseur parmi les alkylamines primaires à chaîne droite en $C_{14}—C_{16}$ et les poly(vinyl)pyrrolidones ayant un poids moléculaire moyen en poids d'au moins 1000.

2. Procédé selon la revendication 1, caractérisé par le fait qu'on choisit une poly(vinyl)pyrrolidone ayant un poids moléculaire moyen en poids entre 10 000 et 100 000.

3. Procédé selon l'une des revendications 1 ou 2, caractérisé par le fait que R'' et R''' sont des radicaux méthyle.

4. Procédé selon l'une des revendications 1 à 3, caractérisé par le fait que l'on emploie une température comprise entre 90° et 130°C.

5. Procédé selon l'une des revendications 1 à 4 caractérisé par le fait que l'on emploie la pression atmosphérique ou superatmosphérique.

6. Procédé selon l'une des revendications 1 à 5, caractérisé par le fait que le catalyseur est présent à raison d'au moins 0,05% en poids par rapport au poids du composé arylé de formule II.

7. Procédé selon l'une des revendications 1 à 6, caractérisé par le fait qu'on utilise comme composé arylé de formule II, le cumène ou le cyclohexylbenzène.

8. Procédé selon l'une des revendication 1 à 7, caractérisé par le fait qu'on utilise comme catalyseur, la 1-hexadécylamine ou la poly(vinyl)pyrrolidone ayant un poids moléculaire moyen en poids de 40 000.

9. Procédé selon l'une des revendications 1 à 8, caractérisé par le fait qu'on utilise comme initiateur, un hydroperoxyde d'aryle substitué par des groupes alkyle ou cycloalkyle.

10. Procédé selon la revendication 9, caractérisé par le fait que l'hydroperoxyde d'aryle est un hydroperoxyde d'aryle de formule I défini à la revendication 1.

11. Procédé selon la revendication 10, caractérisé par le fait que l'initiateur est l'hydroperoxyde de cumène et la composé de formule II défini à la revendication 1 est le cumène.

**Patentansprüche**

1. Verfahren zur Herstellung eines Arylhydroperoxyds der Formel I:

$$Ar—(R—OOH)_n \qquad (I)$$

aus einer entsprechenden Arylverbindung der Formel II:

$$Ar—(R)_m \qquad (II)$$

worin

Ar ein Aryl-Rest und jeder der Reste R unabhängig voneinander Wasserstoff oder ein Rest der Formel

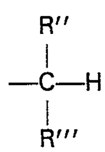 oder 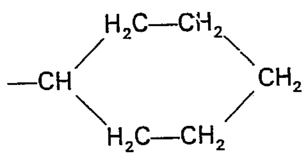

jeder Rest R ein Rest der Formel

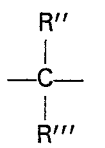 oder 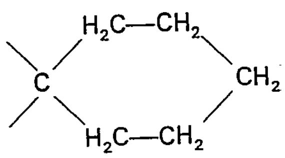

R'' und R''' unabhängig voneinander Wasserstoff oder Alkyl-Reste mit 1 bis 3 Kohlenstoffatom mit der Maßgabe sind, daß nicht beide Reste gleichzeitig Wasserstoff sein können.

m 1 oder 2 mit der Maßgabe ist, daß wenn m 1 ist, R nicht Wasserstoff ist, und wenn m 2 ist, mindestens 1 R nicht Wasserstoff ist, und n 1 oder 2 ist,

wobei die entsprechende Ausgangs-Arylverbindung II mit molekularem Sauerstoff unter Hydroperoxydationsbedingungen in Anwesenheit eines Reaktionsauslösers und eines Katalysators miteinander in Berührung gebracht werden, dadurch gekennzeichnet, daß der Katalysator aus der Gruppe der gradkettigen primären $C_{14}$—$C_{16}$-Alkylamine und Polyvinylpyrrolidone mit einem durchschnittlichen Molekulargewicht von mindestens 1.000 ausgewählt ist.

2. Verfahren gemäß Anspruch 1, dadurch gekennzeichnet, daß das Polyvinylpyrrolidon ein mittleres Molekulargewicht zwischen 10.000 und 100.000 hat.

3. Verfahren gemäß Anspruch 1 oder 2, dadurch gekennzeichnet, daß R'' und R''' Methyl-Reste sind.

4. Verfahren gemäß irgendeinem der Ansprüche 1 bis 3, dadurch gekennzeichnet, daß eine Temperatur im Bereich von 90 bis 130°C angewandt wird.

5. Verfahren gemäß irgendeinem der Ansprüche 1 bis 4, dadurch gekennzeichnet, daß atmosphärischer oder überatmosphärischer Druck angewandt wird.

6. Verfahren gemäß irgendeinem der Ansprüche 1 bis 5, dadurch gekennzeichnet, daß der Katalysator in einer Menge von mindestens 0.5 Gewichtsprozent auf der Basis des Gewichts der Arylverbindung der Formel II anwesend ist.

7. Verfahren gemäß irgendeinem der Ansprüche 1 bis 6, dadurch gekennzeichnet, daß die Arylverbindung der Formel II Cumol oder Cyclohexylbenzol ist.

8. Verfahren gemäß irgendeinem der Ansprüche 1 bis 7, dadurch gekennzeichnet, daß der Katalysator 1-Hexadecylamin oder Polyvinylpyrrolidon mit einem durchschnittlichen Molekulargewicht von 40.000 ist.

9. Verfahren gemäß irgendeinem der Ansprüche 1 bis 8, dadurch gekennzeichnet, daß der Reaktionsauslöser ein Alkyl- oder Cycloalkyl-substituiertes Arylhydroperoxyde ist.

10. Verfahren gemäß Anspruch 9, dadurch gekennzeichnet, daß das eingesetzte Arylhydroperoxid ein Arylhydroperoxid der in Anspruch 1 angegebenen Formel I ist.

11. Verfahren gemäß Anspruch 10, dadurch gekennzeichnet, daß der Reaktionsauslöser Cumolhydroperoxid ist und die Verbindung der im Anspruch 1 angegebenen Formel II Cumol entspricht.